# EUROPEAN PATENT SPECIFICATION

(11) **EP 1 688 112 B1**
(45) Date of publication and mention of the grant of the patent: **14.10.2009**
(21) Application number: 05258111.3
(22) Date of filing: 30.12.2005
(51) Int. Cl.: A61F 13/26, A61F 13/32

(54) **Applicator for feminine hygiene tampon and feminine hygiene product**
Applikator für Tampon für die weibliche Hygiene und Frauenhygieneartikel
Applicateur pour tampon pour l'hygiène féminine et produit d'hygiene feminine

(30) Priority: 31.01.2005 JP 2005024284
(43) Date of publication of application: 09.08.2006
(73) Proprietor: UNI-CHARM CORPORATION, Shikokuchuo-shi, Ehime 799-0111 (JP)
(72) Inventor: Suga, Ayami, c/o Technical Center, Kanonji-shi, Kagawa-ken 769-1602 (JP); Kondo, Hideki, c/o Technical Center, Kanonji-shi, Kagawa-ken 769-1602 (JP)
(74) Representative: Fitchett, Stuart Paul

(56) References cited:
- EP-A- 0 336 529
- EP-A- 0 440 233
- US-A- 2 222 088
- US-A- 3 765 416

## Description

This application is based on and claims the benefit of priority from Japanese Patent Application No. 2005-024284, filed on 31 January 2005

### BACKGROUND OF THE INVENTION

### Field of the Invention

The present invention relates to an applicator for use in inserting a feminine hygiene tampon (hereinafter also simply called a tampon) into a vagina, relates in particular to an applicator for a feminine hygiene tampon which can avoid a push rod of the applicator from remaining in the vagina, and relates in particular to a feminine-hygiene product having the applicator.

### Related Art

Conventionally, for feminine-hygiene products, a feminine-hygiene napkin and tampon are generally known. The feminine-hygiene tampon is an absorbing material which is inserted into a vagina to absorb and hold bodily fluids such as blood. Generally, feminine-hygiene tampons which are commercially available are accommodated in an applicator for use in inserting it into a vagina. Here, the applicator for the feminine-hygiene tampon is formed with a casing which accommodates the tampon and a push rod which is disposed inside the casing and is used to push the tampon out of the casing tip end (see JP-A-61-249464, Patent Reference 1).

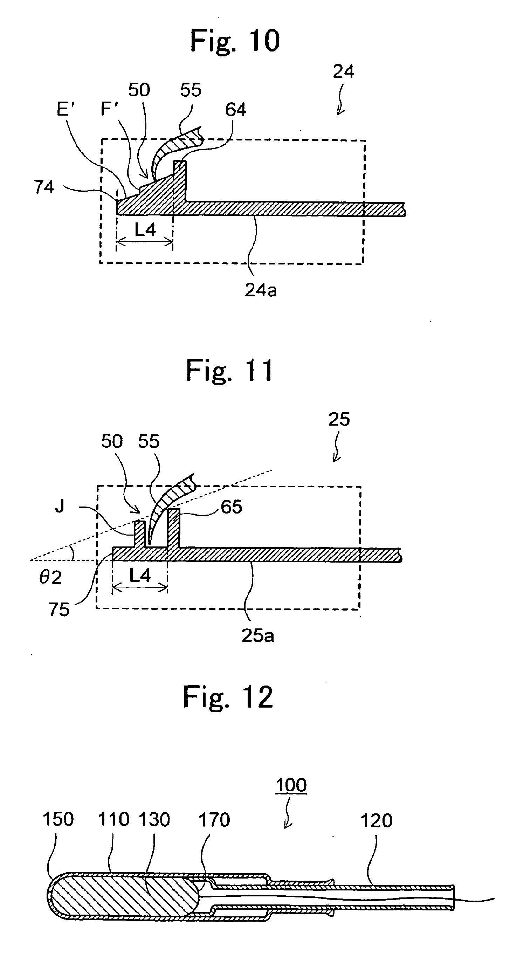

Fig. 12 is a cross section showing an applicator in Patent Reference 1. This applicator 100 has a petaloid tampon outlet 150 at the tip end of a casing 110. A push rod 120 which slides inside the casing 110 pushes an end part of a tampon 130. Thus, the tampon 130 is pushed out of the tampon outlet 150, and is inserted into a vagina.

According to the applicator 100, when the push rod 120 pushes the tampon 130 out of the casing 110 to insert it into a vagina, the push rod 120 is thrust too, causing the petals 150 of the casing 110 to sit at a tip end 170 of the push rod 120. At this time, the force that the petals 150 having opened return to the original state acts on the push rod 120, which triggers the push rod 120 to come out of the tip end 150 of the casing 110. Consequently, a problem sometimes arises that the push rod 120 remains inside the body (in the vagina) even though the casing 110 is taken out.

For a method to solve the problem of the push rod remaining inside the body, for example, a method may be mentioned in which after the push rod is set in the casing during assembly, the end part of the push rod that does not abut against the tampon is extended in diameter, and thus the push rod is prevented from entering the casing.

However, according to this method, since process steps for extending the end part of the push rod in diameter are increased in production, productivity is decreased.

For another method to prevent the push rod from coming out, adopting a push rod with a flange (stopper) may be mentioned (see JP-UM-A-1-072219, Patent Reference 2). Fig. 13 is a cross section showing an applicator in Patent Reference 2. This applicator 101 has a flange (stopper) 161 near a tip end 171 of a push rod 121 that abuts against a tampon 131. Since the stopper 161 is located near the tip end 171 of the push rod 121, even though the push rod 121 is thrust firmly, petals 151 which form a tampon outlet of a casing 111 are less likely to sit at tie flange (stopper) 161, and thus the push rod 121 is less likely to come out of the casing 111.

Further prior art applicators are known from EP 0336529, EP 0440233, EP 3765416 and US 2222088.

US3765416 discloses an applicator comprising a casing which accommodates a tampon and a push rod which is disposed inside the casing to push the tampon out of the casing, wherein the push rod has a flange having a maximum diameter of the push rod at a predetermined distance from a tip end of the push rod abutting against the tampon, and an outer diameter of the push rod from the flange to the tip end is gradually reduced overall.

### SUMMARY OF THE INVENTION

In the applicator 101 described in Patent Reference 2, the outer diameter of the push rod 121 is nearly uniform in the portion from the tip end 171 of the push rod 121 to the flange 161, and the outer diameter of the push rod 121, except the flange 161, is almost the same size.
Thus, a structure is formed in which only the presence of the flange (stopper) 161 contributes to preventing the push rod 121 from coming out and the other parts do not serve to prevent coming out. Therefore, the effect of preventing the push rod from coming out can be obtained to some extent, but it is not perfect, making further improvement desirable.

The present invention has been made in view of these problems. Objects of the invention are to provide an applicator for a feminine hygiene tampon which can reliably prevent a push rod of an applicator from coming out of a casing and remaining inside a vagina, and to provide a feminine hygiene product having the applicator.

In order to solve the problems, the inventors diligently researched as attention was focused on the point that the effect for preventing the push rod from coming out needs to be provided to parts other than the flange (stopper). Consequently, the outer diameter from tip end of the push rod to the flange is formed to gradually increase overall. Thus, when the petals which form the tampon outlet reach the flange, a force that pushes back the push rod into the casing can be exerted on the push rod. As a result, it was found that the push rod can be securely prevented from coming out of the casing. Accordingly, the present invention has been completed. More specifically, the present invention provides the following.

In a first embodiment of the present invention there is provided an applicator for use in inserting a feminine hygiene tampon into a vagina, the applicator comprising: a casing which accommodates a tampon; and a push rod which is disposed inside the casing to push the tampon out of the casing, wherein the push rod has a flange having a maximum diameter of the push rod at a predetermined distance from a tip end of the push rod abutting against the tampon, and an outer diameter of the push rod from the flange to the tip end is reduced overall, characterised in that a portion of the push rod between the flange and the tip end is formed with at least one step.

According to a first embodiment of the present invention, the flange is disposed midway of the push rod, and the outer diameter from the flange to the tip end is reduced overall. Thus, when petals being the tampon outlet (generally meaning the tip ends of the tampon outlet) reach the portion from the tip end of the push rod to the flange, a force that returns the rod into the casing acts on the push rod. Therefore, a force that returns the push rod into the casing also acts on the portion from the tip end to the flange in addition to the flange. Accordingly, the push rod can more reliably be prevented from coming out.

In addition, for "the diameter being reduced overall" in the present invention, it is acceptable that the diameter be reduced overall in appearance, and it is not reduced in diameter continuously. More specifically, the part increased in diameter may partially include a flat part, a stepped part, a recess, etc.

In a second embodiment of the present invention, there is provided an applicator for use in inserting a feminine hygiene tampon into a vagina, the applicator comprising: a casing which accommodates a tampon; and a push rod which is disposed inside the casing to push the tampon out of the casing, wherein the push rod has a flange having a maximum diameter of the push rod at a predetermined distance from a tip end of the push rod abutting against the tampon, and an outer diameter of the push rod from the flange to the tip end is reduced overall, characterised in that a portion of the push rod between the flange and the tip end is formed with a recess and a projection.

According to the second embodiment of the present invention, when the petals forming the tampon outlet reach a wall formed by a step, the petals are caught at the wall formed. At this time, at the top of each wall, a lift is generated that is perpendicular to the force of the petals acting on the top in the direction toward the inside the casing. Therefore, a force that returns the push rod into the casing acts on the push rod, and the push rod can be more reliably prevented from coming out. At the same time, when the petals pass through the recess disposed between the walls, a drop causes vibrations which are transmitted to the petals.
Accordingly, a user of the applicator is made aware that the rod is coming near the insertion limit by the vibrations, and the user can stop pushing the push rod as desired. Therefore, the push rod can be prevented from coming out of the casing.

In a third embodiment of the present invention, an applicator for a feminine hygiene tampon according to the first or second aspects of the present invention is a predetermined distance of 3 mm to 25 mm, inclusive, from the tip end.

According to the third embodiment of the present invention, the distance from the tip end to the flange is 3 mm or more to allow the tip end of the push rod to be protruded out of the petals at the tip end of the casing, and the tampon can be inserted securely. On the other hand, it is 25 mm or less to secure a volume to accommodate the tampon in the casing.

In a fourth embodiment of the present invention, an applicator for a feminine hygiene tampon according to the first aspect of the present invention has a portion from the flange to the tip end which is formed with a stepped slope.

According to the fourth embodiment of the present invention, when the petals forming the tampon outlet reach the portion of the push rod having the stepped slope, in the slope, the force of the petals acts obliquely on the slope. Therefore, a lift is generated that is perpendicular to the force of the petals acting on the slope in the direction toward the inside of the casing. Thus, a force that returns the push rod into the casing acts on the push rod, and the effect can be obtained that prevents the push rod from coming out. At the same time, since a drop in the slope when the petals pass through a step of the stepped portion, vibrations are transmitted to the petals. Accordingly, a user of the applicator is made aware that the rod is coming near the insertion limit by vibrations, and the user can stop pushing the push rod as desired.
Therefore, the push rod can be prevented from coming out of the casing.

According to a fifth embodiment of the present invention, a feminine hygiene product has an applicator for a feminine hygiene tampon according to any one of the first through fifth embodiment of the present invention, and a tampon.

The feminine hygiene product of the fifth embodiment of the present invention has a tampon inside the casing of the applicator. According to the feminine hygiene product of the fourth embodiment of the present invention, it is unlikely that the push rod of the applicator will come out of the casing and remain in the vagina, and the tampon can be inserted into the vagina easily.

According to the present invention, the applicator for a feminine hygiene tampon can more reliably prevent the push rod of the applicator from coming out of the casing and remaining in the vagina, and the feminine hygiene product having the applicator can be provided.

### BRIEF DESCRIPTION OF THE DRAWINGS

Fig. 1 shows a cross section of an applicator of a first example that does not constitute an embodiment of the invention.
Fig_{.} 2 shows an enlarged view of a push rod in Fig. 1.
Fig. 3 shows an enlarged cross section of a modification of the push rod in the first example.
Fig. 4 shows a schematic diagram of the operation of the first example.
Fig. 5 shows a schematic diagram of the operation of the first example.
Fig. 6 shows an enlarged cross section of a push rod of a first embodiment.
Fig. 7 shows an enlarged cross section of a push rod of a second embodiment.
Fig. 8 shows a schematic diagram of the operation of the second embodiment.
Fig. 9 shows a schematic diagram of the operation of the second embodiment.
Fig. 10 shows an enlarged cross section of a modification the push rod of the second embodiment.
Fig. 11 shows an enlarged cross section of a push rod of a third embodiment.
Fig. 12 shows a diagram of an exemplary conventional applicator.
Fig. 13 shows a diagram of another exemplary conventional applicator.

### DETAILED DESCRIPTION OF THE INVENTION

Hereinafter, embodiments of the present invention will be described with reference to the drawings. In addition, in the descriptions of the individual embodiments other than the first embodiment, those in common with those in the first embodiment will be assigned the same numerals and symbols, descriptions thereof will be simplified or omitted.

### First Example

### Overall Form of an Applicator for a Feminine Hygiene Tampon

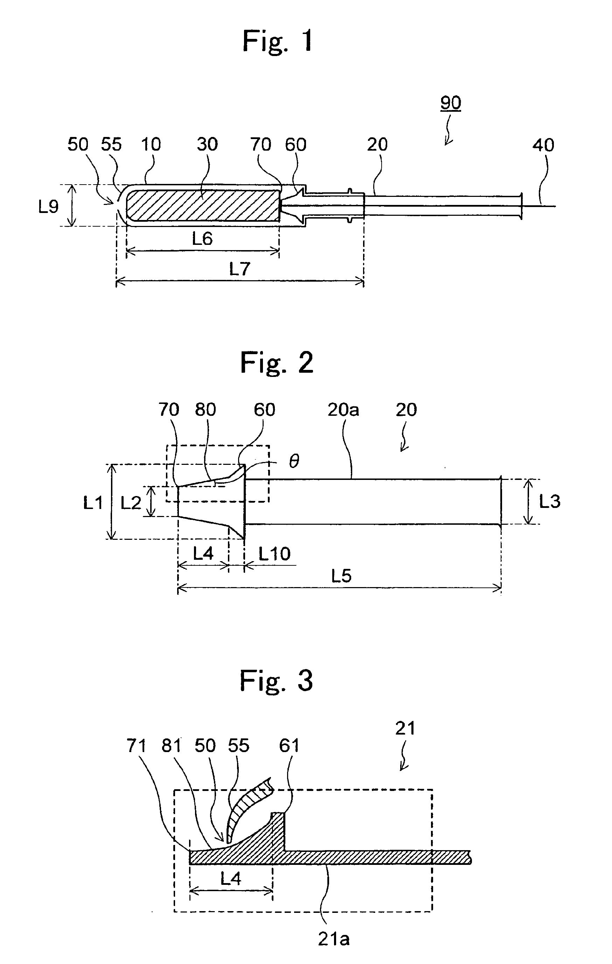

Fig. 1 is a cross section of an applicator for a feminine-hygiene tampon of the first embodiment, which does not constitute an embodiment of the invention. This applicator 90 has a casing 10 which accommodates a tampon 30, and a push rod 20 which is disposed in the casing 10 to push the tampon 30 out of the casing 10. At the tip end of the casing 10, a tampon outlet 50 in a petaloid shape is disposed which is opened in pushing the tampon 30. On the push rod 20, a flange 60 is disposed on the tip end side thereof.

Furthermore, a pull cord 40 is extended from the tampon 30. The inside of the push rod 20 is hollow. The pull cord 40 of the tampon 30 passes through the inside of the push rod 20, and the pull cord 40 is extended to the outside of the applicator from the end of the tampon 30 on the side where the push rod 20 does not abut. Herein after, the individual components of the applicator 90 will be described.

### Casing

The casing 10 has a circular cross section, and a hollow is formed inside to accommodate the tampon 30. At the tip end of the casing 10, the tampon outlet 50 in a petaloid shape is formed which is opened in pushing the tampon 30. At the other end (rear end) of the casing 10, an opening through which the push rod 20 penetrates is disposed.

Preferably, the diameter of the casing 10 (L9 in Fig. 1) generally ranges from 10 mm to 25 mm. Since the tampon to be an absorbing material is accommodated in the casing, an absorbing amount of the tampon accommodated in the casing cannot be secured sufficiently when the diameter is less than 10 mm. Moreover, when the diameter is more than 25 mm, it is not preferable because a user is likely to experience discomfort when inserting the applicator into the vagina or during the use of the tampon.

The casing 10 can be fabricated by injection molding with a thermoplastic such as polyethylene resin. For materials of the casing, materials having flexibility are preferably used for the purpose of providing a bendable function to the petals (slits) which form the tampon outlet 50. Among the materials, polyethylene resins can be used preferably. Furthermore, in order to enhance aesthetic appearance, mica, titanium oxide, pigments, etc., may be added appropriately.

For the casing used for the applicator and the feminine hygiene product according to the present invention, it is acceptable to have a hollow to accommodate a tampon inside, and the shape of the casing is not particularly limited. In order to reduce user discomfort when inserting the applicator into the body as much as possible, it is preferably nearly a cylindrical shape. Moreover, the shape of the opening to be the tampon outlet is also not particularly limited. In order to open bendably, to prevent a tampon from coming out accidentally, and to smoothly insert a tampon into a vagina in pushing the tampon, the petaloid (radial) slit shape is preferable. Push Rod

Fig. 2 is an enlarged view of the push rod 20 shown in Fig. 1. The push rod 20 has a slim cylindrical shape, and has a hollow therein to penetrate through the pull cord 40 of the tampon 30. The push rod 20 has a cylindrical base part 20a, a flange 60 which stands vertically from the base part 20a and has the maximum diameter of the push rod 20, and a beveled part 80 by which the diameter of the push rod 20 is gradually reduced from the flange 60 toward a tip end 70.

Preferably, the length of the push rod 20 in the longitudinal direction (L5 in Fig. 2) is nearly equal to the length of the casing in the longitudinal direction (L7 in Fig. 1) so that the tampon can be pushed into the vagina sufficiently. When the difference between the length of the casing and the length of the push rod is Z (Z = L5 - L7), Z desirably ranges from above -2 mm to below 2 mm (-2 mm < Z < 2 mm). When Z is less than -2 mm, the tampon might not be pushed out enough, whereas when Z is more than 2 mm, the length that the tip end of the push rod is protruded from the casing becomes great, the protruded tip end of the push rod might injure the inside of the vagina.

The flange 60 is a portion that is the maximum diameter of the push rod, and that serves to prevent the push rod from coming out of the tampon outlet in pushing the tampon out of the tampon outlet of the casing, and that also serves as a stopper to prevent the push rod from coming off the opposite end of the tampon outlet.

Since the length of the push rod 20 of the flange 60 in the longitudinal direction (L10 in Fig. 2) requires the strength to the extent that the push rod 20 does not come out or come off the casing 10, it is preferably 1 mm or more.

Furthermore, the diameter of the flange 60 (L1 in Fig. 2), to be the maximum diameter of the push rod 20, is preferably almost the same diameter as that of the casing in order to prevent the push rod from coming off the opposite end of the tampon outlet of the casing. The difference between it and the diameter of the casing is preferably about 2 mm in general. For example, the diameter of the casing (L9 in Fig. 1) is set to range from 10 mm to 25 mm, the diameter of the flange of the push rod (L1 in Fig. 2) preferably ranges from 8 mm to 23 mm, corresponding to the casing. Moreover, the diameter of the base part 20a of the push rod (L3 in Fig. 2) preferably ranges from 4 mm to 20 mm.

The tip end 70 on the side abutting against the tampon 30 has an outer diameter smaller than the outer diameter of the flange 60. The diameter of the tip end of the push rod 70 (L2 in Fig. 2) for use in the present invention preferably ranges from 4 mm to 23 mm. When the diameter of the tip end is less than 4 mm, this is not preferable because the inside of the vagina might be injured in the case in which the tip end contacts the inside of the vagina.

### Beveled Part of the Push Rod

The portion from the tip end 70 of the push rod 20 to the flange 60 has the beveled part 80 by which the diameter of the push rod 20 is gradually increased. The length from the tip end 70 to the flange 60 in the longitudinal direction (L4 in Fig. 2) being "a predetermined distance" in the push rod 20 is preferably 3 mm to 25 mm, inclusive. Generally, the length of the vagina is 80 to 100 mm. Since the portion with sphincters that feels discomfort extends about 40 to 50 mm from the vaginal opening, in consideration of these circumstances, the length of a tampon (L6 in Fig. 1) is preferably about 30 mm to 50 mm. Although the length from the tip end of the push rod to the flange in the longitudinal direction (L4 in Fig. 2) can be selected in accordance with the size of a tampon, for example, when the length of the tampon (L6 in Fig. 1) is 30 mm at minimum, the length from the tip end of the push rod to the flange in the longitudinal direction (L4 in Fig. 2) preferably ranges from 3 to 20 mm.

A beveled angle of the push rod 20 in the longitudinal direction (8 in Fig. 2) ranges from an angle of 3 degrees to 70 degrees, inclusive. For example, when the diameter of the tampon ranges from 10 mm to 15 mm, the length thereof ranges from 40 mm to 50 mm, the length from the tip end of the push rod to the flange in the longitudinal direction (L4 in Fig. 2) ranges from 4 mm to 5 mm, the diameter of the push rod (L1 in Fig. 2) ranges from 10 mm to 15 mm, and the diameter of the tip end (L2 in Fig. 2) ranges from 8 mm to 11 mm, the beveled angle preferably ranges from an angle of 3 degrees to 5 degrees.
Furthermore, the length from the tip end of the push rod to the flange in the longitudinal direction (L4 in Fig. 2) is 3 mm, the diameter of the push rod (L1 in Fig. 2) is 23 mm, and the diameter of the tip end (L2 in Fig. 2) is 4 mm, the beveled angle is an angle of about 70 degrees. Modification of the Beveled Part

Fig. 3 is a partially enlarged cross section of a modification of the push rod (corresponding to the area surrounded by the dotted line in Fig. 2). A push rod 21 of the modification has a cylindrical base part 21a, a flange 61 which stands vertically from the base part 21a and has the maximum diameter of the push rod 21, and a beveled part 81 by which the diameter of the push rod 21 is gradually reduced from the flange 61 toward a tip end 71. The beveled part 81 is different from that in Fig. 2 in that the beveled angle has a curved surface, not a constant slope. As described above, the beveled part may be a curved surface with a recess, or may be a curved surface with a projection.

### Materials and Shapes of the Push Rod

The push rod may be of the same material as that of the casing, or may be of a different material. For example, a thermoplastic such as polyethylene resin is used for fabrication by injection molding. Since forth pushes a tampon out of the casing to insert it into the body, a hard material such as polypropylene and polyester resin can be used. Furthermore, in order to enhance aesthetic appearance, mica, titanium oxide, pigments, etc., can be added as appropriate.

In the push rod according to the present invention, the other end (rear end) projected outside the applicator is pressed, and the push rod slides inside the casing to push the tampon that is accommodated inside the casing out of the tampon outlet of the casing. Therefore, the shape is not particularly limited as long as these functions are satisfied. Furthermore, the inside may or may not be hollow, but hollow is preferable because the pull cord of the tampon can penetrate therethrough.

### Tampon

The shape of the tampon 30 is not particularly restricted as long as it can be accommodated in the casing, but it is preferably a nearly cylindrical shape for the purpose of reducing discomfort as much as possible when a user uses the tampon. Generally, the length of the vagina is 80 to 100 mm. Since the portion with sphincters that fells discomfort extends about 40 to 50 mm from the vagina opening, in consideration of these circumstances, the length of a tampon (L6 in Fig. 1) is preferably about 30 mm to 50 mm.

The material of the tampon 30 is not particularly restricted as long as the material can be formed an absorbing material to absorb and hold bodily fluids. For example, it can form a lump of fibers mainly formed of rayon and cotton having water absorbing properties. Furthermore, in order to prevent fibers from coming off and to reduce discomfort when a user uses a tampon, a surfacing material can be used on the surface of a tampon. For the surfacing material, the following may be mentioned: nonwoven fabric sheets formed of hydrophilic fibers and synthetic fibers such as rayon and cotton, which are fabricated by span lacing (nonwoven fabric formation by high-pressure water flow entangling), a through-air method, and thermal embossing, and perforated films and perforated nets having materials of polyethylene resins. For the tampon for use in the present invention, for example, such a tampon is mentioned in which a lump of fibers mainly formed of rayon and cotton having water absorbing properties is covered with a nonwoven fabric having polyester fibers on the surface thereof by spanbonding, and a pull cord is provided. Pull Cord

The pull cord 40 is a cord for use in pulling the tampon out of the body. For the material of the pull cord, it is not particularly limited as long as it has the strength so that the tampon can be pulled out. For example, cotton yarn, and intertwined yarn of cotton yarn and polyester yarn can be mentioned.

For a method of providing the pull cord to the tampon for use in the present invention, for example, the following method may be mentioned: cotton yarn as the pull cord is sewn to the tampon having a lump of fibers wrapped with a surfacing material by a sewing machine. Furthermore, as another method, the following method can be mentioned: a lump of fibers wrapped with a surfacing material is perforated, the pull cord passes through the hole, and then the end part of the cord is tied. Moreover, when a lump of wide fibers is used, the end part of the pull cord is tied to form a loop, a lump of wide fibers passes through the loop, and then the lump of fibers is folded to wind the cord.

### Method of Fabricating a Feminine Hygiene Product Having the Applicator and the Tampon

An exemplary method of fabricating a feminine hygiene product having the applicator and the tampon of the invention is shown below. The petals 55 which form the tampon outlet 50 of the casing 10 of the applicator are opened along the outer diameter of the casing 10 before thermoforming. The petals 55 are thermoformed, rounded and then closed. Therefore, the push rod 20 is first inserted into the casing 10 from the opening which forms the tampon outlet 50 before thermoforming, from the rear end without the flange 60. Subsequently, the tampon 30 is inserted into the casing 10 as the pull cord 40 penetrates through a hollow inside the push rod 20. Finally, the petals 55 of the tampon outlet 50 are formed round by thermoforming, and the push rod 20 and the tampon 30 are put inside the casing 10. In this manner, the applicator can be fabricated.

### Operation

Next, the operation of the applicator of the first example will be described. First, the rear end of the push rod is pushed in the initial state in Fig. 1, and then the push rod 20 slides inside the casing 10. The tip end 70 of the push rod 20 abuts against the tampon 30 that is accommodated inside the casing 10. The tip end 70 of the push rod 20 pushes the tampon 30 to open the petals 55 of the tampon outlet 50 of the casing 10, and the tampon 30 is pushed out of the tampon outlet 50.

Here, suppose the push rod 20 is further pushed, the tip end of the push rod 20 is protruded through the tampon outlet 50, and the tip ends of the petals 55 are sit at near the tip end of the push rod 20 (the state shown in Fig. 3).

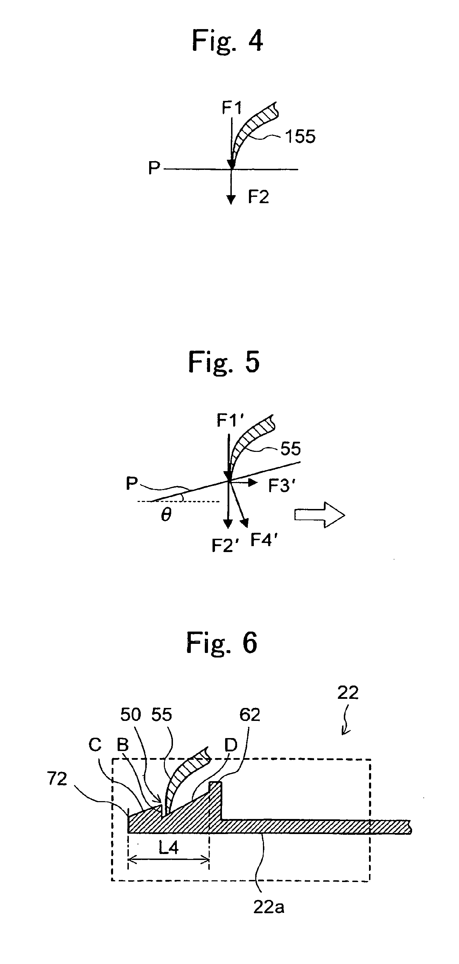

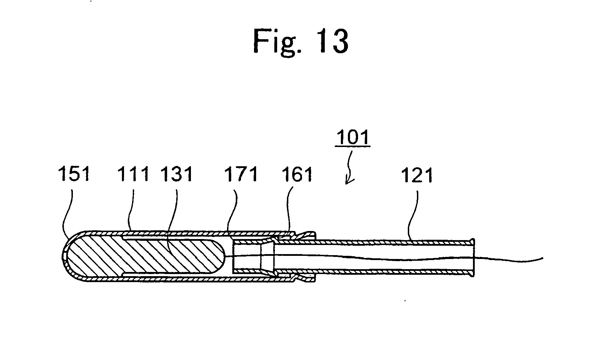

At this time, in the conventional push rod 121 as shown in Fig. 13, the portion from the tip end 171 to the flange 161 is flat with no bevel. In this case, as shown in Fig. 4, when the petals 155 which form the tampon outlet (the tampon outlet 151 in Fig. 13) sit at the portion from the tip end of the push rod to the flange, the force from the tip ends of the petals 155 of the casing act in the direction of F1 which is perpendicular to a plane P corresponding to that portion of the push rod (corresponding to the flat part). At this time, a drag is generated against the force F1 in the plane P of the portion from the tip end of the push rod to the flange. However, since this drag acts perpendicular to the plane P in the direction opposite to a force F2 and is then cancelled, the push rod is not affected in the lateral direction..

On the other hand, in the examples of the present invention, for example, as shown in Figs. 2 and 3, the push rods 20 and 21 have the beveled parts 80 and 81, respectively, from the tip end to the flange. In this case, as shown in Fig. 5, since a plane P in the beveled part of the push rod (corresponding to the beveled parts 80 and 81) has a bevel. Therefore, when the petals 55 forming the tampon outlet (the tampon outlet 50 in Fig. 2) sit at the portion from the tip end of the push rod to the flange, a force F2' and a lift F3' are generated from a force F1' from the tip ends of the petals 55 of the casing, and the total resultant force becomes a force F4'. At this time, since the hardness of the push rod is high, a force that acts against the force F2' is exerted for buffering, and consequently only the lift F3' appears. Then, a force that maintains balance against the lift F3' causes the push rod to return to the right side (an arrow in the drawing) for balancing. Thus, the push rod tends to return to the right side, and the push rod can be prevented from coming out.
In addition, the greater angle θ becomes, the greater force F3' becomes. Therefore, the push rod tends to return to the right side.

Ideally, to the operation like this, friction to hamper the motion between the petals 55 of the tampon outlet 50 and the plane P is little or nothing. Therefore, for the materials of the petals 55 of the tampon outlet 50 and the beveled part, materials with low friction such as plastics are more likely to exert effects. In addition, the operation described is assumed that a user releases the push rod.

### Embodiments

The push rod for use in the present invention is formed to have the outer diameter of the tip end smaller than the outer diameter of the flange, and the outer diameter from the tip end to the flange is gradually increased overall. More specifically, the portion from the tip end to the flange has irregularities with a step, etc. Furthermore, when a step is provided, not only a single step but also multiple steps may be provided, or bumps and dips may be formed.

### First Embodiment

Fig. 6 is a partially enlarged cross section of a push rod of a first embodiment according to the present invention (corresponding to the area surrounded by a dotted line in Fig. 2).

A push rod 22 shown in Fig. 6 has a cylindrical base part 22a, a flange 62 which stands perpendicularly from the base part 22a and has the maximum diameter of the push rod 22, and a slope D, a step B, and a slope C are sequentially formed in stepped fashion from the flange 62 to a tip end 72. The diameter of the push rod 22 is gradually reduced overall toward the tip end 72.

In this case, in addition to the operation and advantages of the first example, the advantages below are further provided. More specifically, even though the petals 55 step over the first slope C, the step B causes vibrations, 'clicks,' to a user. Thus, the user is likely to recognize that that point is the insertion limit, and the user will not put the push rod further. Even if the push rod is further pushed, the presence of the second slope D and the wall of the flange 62 prevent the push rod from being pushed hard as the rod extends over them.
Therefore, the push rod can be effectively prevented from coming off the casing and remaining in the body.

### Second Embodiment

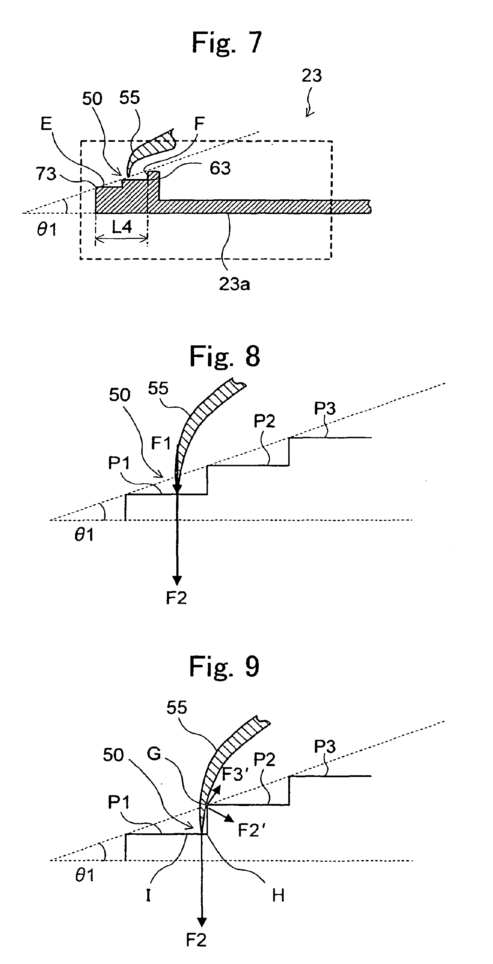

Fig. 7 is a partially enlarged cross section of a push rod of a second embodiment according to the present invention (corresponding to the area surrounded by a dotted line in Fig. 2).

A push rod 23 shown in Fig. 7 has a cylindrical base part 23a, a flange 63 which stands perpendicularly from the base part 23a and has the maximum diameter of the push rod 23, and a flat part E and a step F are continuously formed from the flange 63 toward a tip end 73. The diameter of the push rod 23 is reduced toward the tip end 73 step by step. In addition, in this case, a bevel angel corresponding to θ in Figs. 2 and 5 is θ1. Further, the length from the tip end 73 to the flange 63 in the longitudinal direction being "a predetermined distance" is L4

In this case, as shown in Fig. 8, in planes P1, P2, and P3 corresponding to a flat part E, a force from petals 55 of a tampon outlet 50 acts as a force F1 against the plane of the portion from the tip end of the push rod to the flange as similar to Fig. 4. In this case, since the force F1 acts only vertically to the plane P in the direction of a force F2, the push rod is not affected in the lateral direction in the planes P1, P2, and P3.

However, as shown in Fig. 9, at a corner G of the step, the petals 55 contact with the corner of the wall to generate a force F2', and a lift F3' in the orthogonal direction thereof. In order to keep the lift F3' in balance, the push rod is going to move to the rear end. Therefore, when a user pushes the push rod, the petals 55 reach a point H and then the user releases the push rod from the hand, and the push rod returns on the rear end side. Thus, a behaviour is shown that the positions of the petals 55 return to a point I. More specifically, the push rod is unlikely to come out of the casing. Further, the action occurs strongly that the petals 55 are stopped by a single wall. Therefore, when the petals 55 pass over the wall and then the petals 55 contact with the next wall hard, vibrations, "clicks," can be generated along with the sound of contacting the push rod with the petal. Modification of the Second Embodiment

A push rod 24 shown in Fig. 10 has a cylindrical base part 24a, a flange 64 which stands perpendicularly from the base part 24a and has the maximum diameter of the push rod 24, and a beveled part E' and a step F' are continuously formed from the flange 64 toward tip end 74. The diameter of the push rod 24 is reduced toward the tip end 74 step by step. More specifically, it is different from that shown in Fig. 7 in that the flat part of the step shown in Fig. 7 is a slope. As described above, the rod may have a sloping step.

### Third Embodiment

Fig. 11 is a partially enlarged cross section of a push rod of a third embodiments of the invention (corresponding to the area surrounded by a dotted line in Fig. 2).

A push rod 25 shown in Fig. 11 has a cylindrical base part 25a, a flange 65 which stands perpendicularly from the base part 25a and has the maximum diameter of the push rod 25, and a projection J which is next to the flange 65 and lower than the flange 65. The rod has recesses and projections overall while the diameter thereof is gradually reduced toward a tip end 75. In addition, in this case, a beveled angle corresponding to θ in Figs. 2 and 5 is θ2.

In this case, since the wall is designed to gradually increase in height from the push rod tip end side, the rod is caught at the projection J in the state in which the petals 55 open slightly, and the rod drops in the recess even though the petals 55 pass over the projection J
Consequently, the petals 55 of the casing will not pass over the flange 65 of the push rod, and thus the push rod can be prevented from coming out of the casing and remaining in the body.

While preferred embodiments of the present invention have been described and illustrated above, it is to be understood that they are exemplary of the invention and are not to be considered to be limited. Accordingly, the invention is not to be considered to be limited by the foregoing description and is only limited by the scope of the appended claims.

## Claims

1. An applicator for use in inserting a feminine hygiene tampon into a vagina, the applicator comprising:
a casing (10) which accommodates a tampon; and
a push rod (20) which is disposed inside the casing to push the tampon out of the casing,
wherein the push rod has a flange (60) having a maximum diameter of the push rod at a predetermined distance (L4) from a tip end (70) of the push rod abutting against the tampon, and
an outer diameter of the push rod from the flange to the tip end its reduced overall
**characterised in that** a portion of the push rod between the flange and the tip end is formed with at least one step.

2. An applicator for use in inserting a feminine hygiene tampon into a vagina, the applicator comprising:
a casing (10) which accommodates a tampon; and
a push rod (20) which is disposed inside the casing to push the tampon out of the casing,
wherein the push rod has a flange (60) having a maximum diameter of the at a predetermined distance (L4) from a tip end (70) of the push rod abutting against the tampon, and
an outer diameter of the push rod from the flange to the tip end is reduced overall,
**characterised in that** a portion of the push rod between the flange and the tip end is formed with a recess and a projection (J).

3. The applicator for a feminine hygiene tampon according to claim 1 or 2, wherein the predetermined distance is 3 mm to 25 mm, inclusive, from the tip end.

4. The applicator for a feminine hygiene tampon according to claim 1, wherein a portion from the flange to the tip end is formed with a stepped slope.

5. The applicator for a feminine hygiene tampon according to claim 1or 2, wherein the outer diameter of the push rod, between the flange and the tip end, is formed to cause vibrations between the push rod and an opening of the casing, when in use.

6. A feminine hygiene product comprising:
an applicator for a feminine hygiene tampon according to any one of claims 1 to 5; and
a tampon.

## Patentansprüche

1. Applikator zum Einführen eines Frauenhygiene-Tampons in die Vagina, umfassend:
ein Gehäuse (10) zur Aufnahme des Tampons; und
eine im Gehäuse angeordnete Schubstange (20) zum Herausschieben des Tampons aus dem Gehäuse,
wobei die Schubstange einen Flansch (60) mit einem maximalen Schubstangen-Durchmesser mit einem vorgegebenen Abstand (L4) von einem gegen den Tampon stoßenden Spitzenende (70) der Schubstange aufweist, und
wobei ein Außendurchmesser der Schubstange vom Flansch zum Spitzenende hin ganzheitlich abnimmt,
**dadurch gekennzeichnet, dass** ein Abschnitt der Schubstange zwischen dem Flansch und dem Spitzenende mindestens eine Stufe aufweist.

2. Applikator zum Einführen eines Frauenhygiene-Tampons in die Vagina, umfassend:
ein Gehäuse (10) zur Aufnahme des Tampons; und
eine im Gehäuse angeordnete Schubstange (20) zum Herausschieben des Tampons aus dem Gehäuse,
wobei die Schubstange einen Flansch (60) mit einem maximalen Schubstangen-Durchmesser mit einem vorgegebenen Abstand (L4) von einem gegen den Tampon stoßenden Spitzenende (70) der Schubstange aufweist, und
wobei ein Außendurchmesser der Schubstange vom Flansch zum Spitzenende hin ganzheitlich abnimmt,
**dadurch gekennzeichnet, dass** ein Abschnitt der Schubstange zwischen dem Flansch und dem Spitzenende eine Ausnehmung und einen Vorsprung (J) aufweist.

3. Applikator für einen Frauenhygiene-Tampon nach Anspruch 1 oder 2, wobei der vorgegebene Abstand vom Spitzenende 3 mm bis einschließlich 25 mm beträgt.

4. Applikator für einen Frauenhygiene-Tampon nach Anspruch 1, wobei ein Abschnitt zwischen dem Flansch und dem Spitzenende eine abgestufte Schräge aufweist.

5. Applikator für einen Frauenhygiene-Tampon nach Anspruch 1 oder 2, wobei der Außendurchmesser der Schubstange zwischen dem Flansch und dem Spitzenende so ausgebildet ist, dass er beim Gebrauch Vibrationen zwischen der Schubstange und einer Öffnung des Gehäuses verursacht.

6. Frauenhygiene-Produkt umfassend:
einen Applikator für einen Frauenhygiene-Tampon nach einem der Ansprüche 1 bis 5 und einen Tampon.

## Revendications

1. Applicateur destiné à être utilisé pour insérer un tampon d'hygiène féminine dans un vagin, l'applicateur comportant :
une enveloppe (10) qui contient un tampon ; et
une tige poussoir (20) qui est disposée à l'intérieur de l'enveloppe pour pousser le tampon hors de l'enveloppe,
dans lequel la tige poussoir a une collerette (60) ayant un diamètre maximum de la tige poussoir à une distance prédéterminée (L4) d'une extrémité de pointe (70) de la tige poussoir venant en butée contre le tampon, et
un diamètre extérieur de la tige poussoir allant de la collerette à l'extrémité de pointe est réduit dans son ensemble,
**caractérisé en ce qu'**une partie de la tige poussoir entre la collerette et l'extrémité de pointe est formée avec au moins un gradin.

2. Applicateur destiné à être utilisé pour insérer un tampon d'hygiène féminine dans un vagin, l'applicateur comportant :
une enveloppe (10) qui contient un tampon ; et
une tige poussoir (20) qui est disposée à l'intérieur de l'enveloppe pour pousser le tampon hors de l'enveloppe,
dans lequel la tige poussoir a une collerette (60) ayant un diamètre maximum de la tige poussoir à une distance prédéterminée (L4) d'une extrémité de pointe (70) de la tige poussoir venant en butée contre le tampon, et
un diamètre extérieur de la tige poussoir allant de la collerette à l'extrémité de pointe est réduit dans son ensemble,
**caractérisé en ce qu'**une partie de la tige poussoir entre la collerette et l'extrémité de pointe est formée avec un évidement et une partie saillante (J).

3. Applicateur pour tampon d'hygiène féminine selon la revendication 1 ou la revendication 2, dans lequel la distance prédéterminée est de 3 mm à 25 mm, compris, depuis l'extrémité de pointe.

4. Applicateur pour tampon d'hygiène féminine selon la revendication 1, dans lequel une partie allant de la collerette à l'extrémité de pointe est formée avec une inclinaison en gradins.

5. Applicateur pour tampon d'hygiène féminine selon la revendication 1 ou la revendication 2, dans lequel le diamètre extérieur de la tige poussoir, entre la collerette et l'extrémité de pointe, est formé pour entraîner des vibrations entre la tige poussoir et une ouverture de l'enveloppe, lors de l'utilisation.

6. Produit d'hygiène féminine comportant :
un applicateur pour tampon d'hygiène féminine selon l'une quelconque des revendications 1 à 5 ; et
un tampon.
